# EUROPEAN PATENT APPLICATION

(11) **EP 3 854 352 A1**
(43) Date of publication of application: **28.07.2021**
(21) Application number: 20153762.8
(22) Date of filing: 27.01.2020
(51) Int. Cl.: A61F 2/24, A61F 2/962, A61F 2/95

(54) **DELIVERY SYSTEM FOR A SELF-EXPANDING STRUCTURE COMPRISING AN IMPROVED COOLING MECHANISM THEREFOR**

(71) Applicant: Biotronik AG, 8180 Bülach (CH)
(72) Inventor: NOLLERT, Georg, 82064 Strasslach (DE); KISSLING, Tobias, 3005 Bern (CH)
(74) Representative: Galander, Marcus

(57) **Abstract**

The invention relates to a delivery system (100) adapted for delivery and for deployment of a self-expanding structure such as a stent (200) or a heart valve prosthesis, comprising an inner shaft (110) and an outer shaft (120), optionally further comprising a capsule (121), arranged around the inner shaft (110), the delivery system (100) being configured to receive a self-expanding structure (200) between the inner shaft (110) and the outer shaft (120) and/or the capsule (121) in a compressed / crimped state, and a cooling means (300) for cooling the self-expanding structure (200) to a desired temperature, thereby facilitating recapture of the self-expanding structure (200) between the inner shaft (110) and the outer shaft (120) and/or the capsule (121), wherein the delivery system (100) comprises a connecting means (140) for releasably fixing the self-expanding structure (200) to the delivery system (100) wherein the connecting means (140) itself is coolable by the cooling means (300) such as a cooling device.

## Description

The present invention relates to a delivery system for a self-expanding structure such as a catheter for a self-expanding implant, the delivery system comprising a cooling mechanism for the self-expanding structure. The delivery system may be, e.g., used for the delivery and deployment of a self-expanding implant such as a stent or a heart valve prosthesis in a human or animal body. However, the present invention also relates to a delivery system comprising a cooling mechanism for a non-implantable self-expanding structure such as a positioning aid, or a connector arm, or a connector feeler, or the like.

The present invention further relates to an assembly comprising the delivery system and a self-expanding structure such as a stent or a heart valve prosthesis.

The present invention particularly relates to a catheter comprising a cooling means for a self-expanding artificial aortic heart valve, the aortic heart valve comprising a frame structure such as a self-expanding stent.

Specifically, the invention relates to a delivery system (100) for delivery and deployment of a self-expanding structure such as a stent (200) or an aortic heart valve prosthesis, the delivery system (100) comprising an inner shaft (110) and an outer shaft (120) arranged around the inner shaft (110), optionally further comprising a capsule (121) at a distal end of the outer shaft (120), and being arranged around the inner shaft (110), the delivery system (100) being configured to receive a self-expanding structure (200) between the inner shaft (110) and the outer shaft (120) and/or the capsule (121) in a compressed / crimped state, the delivery system (100) further comprising a cooling means (300) for cooling the self-expanding structure (200) to a desired temperature, thereby facilitating recapture of the self-expanding structure (200) between the inner shaft (110) and the outer shaft (120) and/or the capsule (121),
wherein the delivery system (100) further comprises a connecting means (140) for releasably fixing the self-expanding structure (200) to the delivery system (100), wherein the connecting means (140) itself is coolable by the cooling means (300) such as a cooling device.

Stents are medical implants which are commonly used to dilate constricted vessels or hollow organs, e.g., blood vessels. In addition, artificial heart valve implants for replacing a diseased natural heart valve often contain a stent or some sort of a support structure / frame structure to which valve leaflets or cusps are coupled. The stent or support structure is typically delivered to its target site in the human or animal body in a radially compressed state inside of a catheter.

Self-expanding structures such as self-expanding stents or heart valve prostheses assume their radially expanded state automatically when released by a delivery system such as a catheter, but without having additional dilating means such as an expandable balloon or any other suitable force-expandable means. Such self-expanding structures (e.g. a self-expanding stent or a self-expanding heart valve prosthesis) are normally made of a shape-memory material such as nitinol. Nitinol is an intermetallic compound containing nickel and titanium atoms and it assumes an austenitic state at higher temperature and a martensitic state at lower temperature. For instance, a nitinol stent is typically manufactured at high temperatures, where nitinol is in the austenitic state. To load such a stent onto a catheter for delivery, the stent is typically cooled down to the martensitic state, where it can be plastically deformed, and is loaded onto an inner shaft of the catheter thereby transforming into a crimped state and hold in such a compressed state with the aid of a retention means of the catheter such as an outer shaft optionally having a distal capsule structure. The cooling reduces strain to the material and facilitates loading. At physiological temperatures in the human or animal body, the nitinol material then reassumes its original shape (i.e. a shape memory material) and displays superelastic properties which are important to provide a constant chronic outward force (COF) to a vessel or hollow organ, e.g., an aortic annulus plane of a patient's heart into which it is implanted.

For instance, during an implantation procedure of a self-expanding vascular stent, the surgeon eventually has to reposition the self-expanding structure one to, e.g., three times in order to achieve optimal placement in the vessel. To this end, the stent is recaptured in the catheter and released again for repositioning. Since the temperature in the patient body is too high to plastically deform the shape-memory material of the stent, damage is often inflicted on the stent during such recapture events, reducing the chronic outward force of the implanted stent. This may lead to complications, such as paravalvular leakage (in case of heart valve stents) or shifting (embolization) of the stent in the vessel. Accordingly, only a small number of recapture events is tolerated (typically two to maximally three recapture events), and the stent must be removed and replaced if this number is exceeded, leading to an increased duration of the catheterization procedure and significantly raised costs.

This problem has been addressed in the prior art by a cooling means capable of cooling a self-expanding stent inside of the patient body to facilitate recapture and to reduce strain to the stent material during recapture events. This cooling means includes an inflatable balloon configured to contact the inside of the stent in the inflated state, wherein the balloon is filled with a cooling fluid to cool the stent (WO 02/087656). In addition, direct injection of a cooling fluid into the vessel close to the deployed stent in order to cool and recapture the stent has been also described in the prior art (US 2006/0184231).
However, a cooling means incorporating an inflatable balloon is relatively bulky and significantly increases complexity of manufacturing a catheter. On the other hand, directly injecting cooling fluid into the patient body may cause a potential health risk.

Therefore, an objective of the present invention is to provide a delivery system comprising an improved cooling means and a cooling mechanism for a self-expanding structure such as a self-expanding implant. The present invention allows for cooling the self-expanding structure, e.g. during a recapture event, which is however improved in view of the described drawbacks of the prior art. In certain embodiments, for instance, a delivery system in accordance with the invention exhibits a smaller size and makes use of less components compared to the prior art due to the arrangement of the herein disclosed cooling means and the respective cooling mechanism.

This objective is attained by the subject matter of independent claim 1. Advantageous embodiments of the invention are claimed as dependent claims 2 to 15 and are described hereafter. Further embodiments of the invention can be *inter alia* derived from the below consecutively numbered embodiments.

Following the above, in one aspect the invention relates to a delivery system for the delivery and/or deployment of a self-expanding structure such as a stent or a heart valve prosthesis in a human or animal body, the delivery system comprising an inner shaft extending along a longitudinal axis and an outer shaft arranged around the inner shaft along the longitudinal axis, wherein the delivery system is configured to receive a self-expanding structure between the inner shaft and the outer shaft in a compressed / crimped state. Optionally, the delivery system comprises a distal capsule structure with an enlarged diameter in the distal region (typically attached to the outer shaft) to accommodate an implant. The delivery system further comprises a cooling means such as a cooling device for cooling the self-expanding structure (e.g. stent or heart valve prosthesis) to a desired temperature, thereby facilitating recapture of the self-expanding structure between the inner shaft and the outer shaft and/or capsule. The delivery system further comprises a connecting means such as a prosthesis connector and/or a connector arm and/or a connector feeler for fixing the self-expanding structure to the delivery system, particularly to the inner shaft, wherein the connecting means present in the delivery system is coolable itself by the cooling means. For instance, the connecting arm / connecting feeler could comprise a thermal insulation, so to limit heat transfer proximal from the connector.

With the context of the invention, the expression "delivery system" shall be understood as any suitable medical system that allows for the delivery and/or deployment and/or recapture and/or resheathing of any suitable kind of a self-expanding structure, the self-expanding structure particularly being made of a shape memory material such as nitinol.

In certain instances, the expressions "delivery system" and "catheter" may be used interchangeably with the context of the invention.

Following the above, ease of recapture / resheathing of the self-expanding structure is facilitated and damage to the self-expanding structure during recapture / resheathing is prevented in an effective manner using the self-expanding structure connecting means as a heat bridge in accordance with the invention. Thus, the connecting means such a prosthesis connector and/or a connector arm and/or a connector feeler, but not limited thereto, fulfils at least a double function in accordance with the invention, namely (i) fixation of the self-expanding structure to the delivery system, e.g., in a partially deployed state, and (ii) heat conduction. This has the advantage that additional heat-conducting components such as an inflatable balloon filled with cooling fluid are not necessary, which thus reduces size, complexity and production costs of such a delivery system, e.g., a catheter.

In the context of the present specification, the terms "self-expanding structure", "stent", "frame structure", "support structure" or the like, which may be used interchangeably, describe a compressible and self-expanding mesh-like, wire-based, optionally braided, or coil-like structure, but not limited thereto, composed of interconnected strut-like elements forming openings (e.g. also termed cells) between the strut-like elements, wherein the self-expanding structure is configured to provide a radially oriented (in respect of a longitudinal axis of the self-expanding structure) outward force to engage surrounding tissue and tightly anchor the self-expanding structure in the tissue. Particularly, the self-expanding structure being made of a shape memory material such as nitinol.

The self-expanding stent according to the invention may be implanted without additional components (such as, e.g., peripheral stents designed to expand constrictions of peripheral arteries) or may be part of a prothesis, e.g., a heart valve prosthesis, particularly an aortic heart valve prosthesis, for replacing a native heart valve, comprising a valve structure that is connected to the stent.

In view of the above, the skilled person is readily aware that coronary stents typically are balloon-expandable whereas peripheral stents often are self-expanding. Resheathing is typically important for heart valve prostheses, but could be also relevant for medical devices such as LAA occluders, aortic grafts, etc.

In particular, the delivery system extends along the longitudinal axis between a proximal end and a distal end. The distal end is particularly formed by a tip section or nosecone having a tip and is configured to be inserted into the patient vasculature, e.g., into a femoral artery, during catheter-based heart valve replacement such as TAVI, TAVR, or PAVR. Besides aortic, this may also apply to mitral, tricuspid or pulmonal valve replacement in accordance with the invention. The proximal end is designed to point towards the physician during catheterization and typically contains a handle for gripping the catheter and an actuating, optionally steering, mechanism to guide the tip of the catheter through the blood vessels. Further, the handle comprises an actuating mechanism for actuating a release and/or a re-sheathing of a self-expanding structure such as a medical implant, particularly a heart valve prosthesis.

With the context of the invention, the abbreviation "TAVI" denotes transcatheter aortic valve implantation", the abbreviation "TAVR" denotes transcatheter aortic valve replacement, and the abbreviation "PAVR" denotes percutaneous aortic valve replacement.

As used herein, the term "proximal" refers to the direction towards the physician implanting the self-expanding structure or the artificial heart valve comprising a self-expanding structure, and the term "distal" refers to the direction towards the vessel or hollow organ of the patient, e.g., an aorta.

The connecting means of the catheter (e.g. a prosthesis connector and/or a connector arm and/or a connector feeler) is particularly configured to hold the self-expanding structure, e.g., in a partially deployed state, such that it can be recaptured. In other words, the connecting means prevents the self-expanding structure (e.g. an implant) from being completely released from the delivery system, e.g., a catheter. In the partially deployed state, the distal end of the self-expanding structure is expanded and particularly extends towards surrounding tissue (e.g., blood vessel walls), whereas the proximal end of the self-expanding structure is fixed to the connecting means. Furthermore, the connecting means is particularly fixed to the inner shaft. For instance, the connecting means such as a prosthesis connector may comprise a central though-hole, through which the inner shaft extends, the though-hole particularly being sized such that a press-fit connection between the inner shaft and the connector is established. The inner shaft, e.g. hypotube, may also be welded to a connecting means such as a prosthesis connector and further comprise a polymer tube arranged within the inner shaft which provides a guidewire lumen.

Within the context of the present specification, the term "deployment" means implanting the self-expanding structure in a vessel or hollow organ of a human or animal, such that the self-expanding structure such as a stent expands to anchor the self-expanding structure in the wall of the vessel or hollow organ. The term "partially deployed" refers to a configuration where at least a part of the self-expanding structure is in the expanded configuration, wherein another part remains attached to the catheter, particularly the connecting means.

With the context of the present invention, "a connecting means", "a connector arm", "a connector feeler" may be any mechanical means suitable for (releasably) fixing / holding a self-expanding structure such as an implant connected to a delivery system, e.g., to an inner shaft of a catheter. For instance, suitable connecting means in accordance with the invention may be a prosthesis connector with a recess and/or a protrusion for (releasably) fixing / holding an eyelet and/or a tab, respectively, of a self-expanding stent.

Optionally, the prosthesis connector may further comprise a connector arm and/or a connector feeler and/or a connector tether or the like for a controlled release of a self-expanding structure. Alternatively, a connecting means may be a mere connector arm or connector feeler or connector tether itself or combinations thereof without being attached to a prosthesis connector.

To deploy and recapture the self-expanding structure, the inner shaft and the outer shaft are particularly movable relative to one another along the longitudinal axis, i.e., the outer shaft, optionally comprising a distal capsule structure, may be retractable (movable in the proximal direction) to uncover the self-expanding structure, such that the self-expanding structure may self-expand. This motion is typically controlled by an actuator at the handle at the proximal end of a delivery system. The outer shaft may comprise a capsule (typically formed at least partially from a metallic material) which surrounds the self-expanding structure in the undeployed state and is retractable in the proximal direction to uncover the self-expanding structure. The capsule may be movable relative to the inner shaft independently of the remaining outer shaft or together, typically together, with the outer shaft. Of course, the delivery system according to the present invention may contain additional shafts, e.g., for stabilization or as additional guidewire lumen. Typically, one guidewire lumen arranged within inner shaft.

In particular, the cooling means according to the invention is positioned in a handle or actuating portion of the delivery system, particularly at the proximal end. Alternatively, the cooling means may be positioned separately from the delivery system, but being connectable with the delivery system. In this manner, the cooling device may be designed larger and/or reusable. Any cooling mechanism known from the state of the art may be applied to generate cold by the cooling means via a connecting element according to the invention, e.g., vapor compression cooling, vapor absorption cooling, adsorption cooling, diffusion-absorption cooling, steam jet cooling, the Joule-Thomson effect, pulse tube refrigeration, thermoelectric effect, magnetic cooling or evaporation cooling.

The desired temperature is particularly chosen low enough to allow recapture of the self-expanding structure, but high enough to prevent tissue damage. In particular, the self-expanding structure is formed from a shape-memory material (also termed shape-memory alloy), particularly nitinol (a nickel titanium intermetallic compound with a ratio between nickel and titanium of about 40% to 60%). In this case, the desired temperature is particularly below the starting temperature of the martensitic transition. Below this limit, the self-expanding structure can be easily compressed without damage to the material and can therefore be easily recaptured.

In certain embodiments, the connecting means such as a prosthesis connector and/or a connector arm and/or a connector feeler and/or a connector tether comprises at least one recess and/or at least one protrusion configured to be engaged by a connecting element of the self-expanding structure such as a stent to fix the stent to the delivery system, e.g. a catheter, wherein the recess and/or protrusion comprises a contacting surface configured to contact the connecting element when the connecting element engages the recess / protrusion, such that heat is conducted between the connecting element and the contacting surface.

In certain embodiments, the connecting means such as a prosthesis connector and/or a connector arm and/or a connector feeler and/or a connector tether comprises a plurality of recesses and/or protrusions configured to be engaged by respective connecting elements of the self-expanding structure such as a stent to fix the stent to the delivery system, wherein the recesses and/or protrusions each comprise a contacting surface to contact the respective connecting element when the respective connecting element engages the respective recess and/or protrusion, such that heat is conducted between the respective connecting element and the respective contacting surface.

When using a connecting surface within a recess and/or protrusion of a connecting means as a heat bridge to cool the self-expanding structure via its connecting element, a large contact surface, which is important for optimal heat conduction, can be established.

The connecting element of the self-expanding structure is particularly an eyelet or a tab, but not limited thereto.

In certain embodiments, the connecting means comprises a distal end, wherein the at least one recess and/or protrusion extends towards the distal end, such that the self-expanding structure extends from the distal end in the distal direction when the connecting element of the self-expanding structure engages the recess and/or protrusion, wherein the contacting surface is inclined toward said distal end by an inclination angle with respect to the longitudinal axis. In other words, the contacting surface extends radially outside with respect to the longitudinal axis towards said distal end. In particular, the inclination angle is 30° to 60°, wherein more particularly the inclination angle is 45°.

In the partially deployed state, the proximal end of the self-expanding structure with the connecting elements fixed to the connecting means of the delivery system is typically less expanded than the distal end of the self-expanding structure. This could be the other way around for certain delivery systems, especially for a transapical delivery. This results in the connecting elements extending radially outward. By inclining the contact surface in the same manner, the contact area between the contact surfaces and the connecting elements is maximized in the partially deployed state, thereby improving heat conduction between the cooled connecting means and the self-expanding structure. Further benefits to mention may be reducing strain even in delivery systems without cooling system.

In certain embodiments, the contacting surface or the connecting element is at least partially covered with a soft heat-conducting material. For instance, a thermally conductive pad may be used; i.e. a pad made of a heat-conducting material applied to a heatsink.

In computing and electronics, thermal pads (also called thermally conductive pad or thermal interface pad) are a pre-formed square or rectangle of solid material (often paraffin wax or silicone based) commonly found on the underside of heatsinks to aid the conduction of heat away from the component being cooled (such as a CPU or another chip) and into the heatsink (usually made from aluminium or copper). Such may be used in an analogous but adapted manner for the subject-matter of the present invention.

Thermal pads and thermal compounds are used to fill air gaps caused by imperfectly flat or smooth surfaces which should be in thermal contact; they would not be needed between perfectly flat and smooth surfaces. Thermal pads are relatively firm at room temperature, but become soft and well able to fill gaps at higher temperatures.

It is an alternative to thermal grease to be used as thermal interface material.

Thermal grease (also called CPU grease, heat paste, heat sink compound, heat sink paste, thermal compound, thermal gel, thermal interface material, or thermal paste) is a thermally conductive (but usually electrically insulating) compound, which is commonly used as an interface between heat sinks and heat sources such as high-power semiconductor devices.

The main role of thermal grease is to eliminate air gaps or spaces (which act as thermal insulation) from the interface area in order to maximize heat transfer and dissipation. Thermal grease is an example of a thermal interface material, and may be also used in an analogous but adapted manner for the subject-matter of the present invention.

As opposed to thermal adhesive, thermal grease does not add mechanical strength to the bond between heat source and heat sink. It will have to be coupled with a mechanical fixation mechanism such as screws, applying pressure between the two, spreading the thermal grease onto the heat source.

In any event, such a heat conductive material must be a biocompatible and medically acceptable material in accordance with the invention.

This further improves heat conduction between the connecting means and the self-expanding structure.

Advantageously, a heat-conducting material on the connecting means does not permanently remain in the patient body after deployment of the self-expanding structure such as a stent, thus reducing potential health risks.

In an additional or alternative embodiment, the self-expanding structure itself comprises a heat-conducting additional material on, e.g., eyelets / tabs which though would remain in the body, but would be biocompatible for extended duration.

In certain embodiments, the delivery system comprises a cooling element which is in direct thermal contact with the connecting means, such that heat may be directly conducted between the connecting means and the cooling element, wherein the cooling means is configured to cool the cooling element.

Such a cooling element provides efficient cooling by thermal contact with the connecting means while being easily adaptable to different predetermined connector geometries. In other words, the connecting means such as a prosthesis connector, a connector arm, a connector feeler does not have to be redesigned to apply the cooling mechanism.

In an embodiment of the invention, the cooling element and the connecting means may be configured and arranged in such a way to allow for a maximized heat transfer, e.g. through a large contact area or a large contact surface, respectively.

In certain embodiments, the cooling means comprises a cold source configured to cool a cooling fluid and to provide the cooling fluid at the connecting means, such that the connecting means is coolable by the cooling fluid.

In certain embodiments, the cooling means comprises a cold source configured to cool a cooling fluid and to provide the cooling fluid at the cooling element, such that the cooling element is coolable by the cooling fluid.

The cooling fluid itself may be selected from saline, alcohol water solutions, solutions comprising PEG, etc. Preferably, a cooling fluid may be applied which is safe in terms of being physiologically acceptable even in case of an undesirable leakage into the blood stream.

In certain embodiments, the cooling means such as a cooling device comprises a cold source configured to cool a cooling fluid and to provide the cooling fluid at the connecting means and the cooling element, such that the connecting means and the cooling element are coolable by the cooling fluid.

In accordance with the invention, a cooling fluid ensures efficient and easily controllable heat transfer to cool the connecting means and the cooling element.

In certain embodiments, the connecting means and/or the cooling element comprises at least one channel for conducting the cooling fluid, wherein the channel comprises a first opening and a second opening, and wherein the first opening and the second opening are connected to the cold source, such that the cooling fluid is able to flow from the cold source into the first opening, through the channel, and through said second opening, particularly back to the cold source. In particular, the channel extends between the first opening and the second opening.

With the context of the invention, for instance such channels can be configured such that they provide a large contact area for heat transfer, e.g. by having meander-shape or spiral-shape. Such a design of a channel could be inspired, e.g., by laboratory condensers.

For the manufacture of such channels, two half-shell parts could be provided to cut the channels on one or both of the individual parts. The half-shells can then be assembled in a fluid tight manner to provide the channels. Also 3D-printing could be used to provide a cooling element and/or connecting means with channels in accordance with the present invention.

In certain embodiments, additionally or alternatively the connecting means and/or the cooling element comprises at least one separate tubing for conducting the cooling fluid, wherein the tubing comprises a first opening and a second opening, and wherein the first opening and the second opening are connected to the cold source, such that the cooling fluid is able to flow from the cold source into the first opening, through the tubing, and through said second opening, particularly back to the cold source. In particular, the tubing extends between the first opening and the second opening.

In certain embodiments, the cooling means such as a cooling device comprises a pump or a compressor for generating a current of the cooling fluid.

In certain embodiments, the cooling means such as a cooling device comprises at least one conduit for conducting the cooling fluid between the cold source and the channel.

In certain embodiments, the cooling means such as a cooling device comprises at least one tubing for conducting the cooling fluid between the cold source and the channel.

In certain embodiments, the conduit is formed by a lumen of the delivery system. For example, the conduit may be a tubing arranged within the lumen between the inner shaft and the outer shaft of the delivery system; e.g. a catheter.

In certain embodiments, the cold source is comprised in the delivery system. Alternatively, according to certain embodiments, the cold source is a unit separate from the delivery system, but connectable to the catheter.

In certain embodiments, the cooling fluid is a liquid, particularly a salt solution, more particularly a sodium chloride (NaCl) solution.

In particular, the salt solution, particularly the sodium chloride solution, has a concentration of 7 grams/liter to 11 grams/liter, more particularly 8 grams/liter to 10 gram/liter, most particularly 9 grams/liter. In other words, the salt solution is isotonic (i.e. being isoosmotic to human blood plasma; i.e. being physiologically acceptable). This has the advantage that no adverse reaction of the patient's body to the cooling fluid occurs even in the unlikely event if cooling fluid leaves the delivery system and enters the bloodstream.

Alternatively, a salt solution of a higher concentration may be used to be able to cool the cooling fluid to a lower temperature without freezing due to the freezing point reduction effect. This can be of advantage, e.g., for stents having a corresponding low martensitic transition temperature and/or to overcome large heating effects of the bloodstream.

In certain embodiments, the cooling fluid is a gas, particularly N₂O. N₂O is commercially used for cryoablation in catheters. In certain embodiments, the cooling fluid may be selected from alcohol-water (e.g. ethanol), PEG-water, glycerol-water, sugar-water solutions and the like.

In certain embodiments, the cooling means such as a cooling device comprises a thermoelectric element, particularly a Peltier element, in thermal contact with the connecting means or the cooling element, wherein the thermoelectric element is configured to cool the connecting means or the cooling element.

Thermoelectric elements operate by the thermoelectric effect and comprise a first and a second side. When a DC electric current is applied to the device, it transports heat from the first side to the second side, so that the first side gets cooler while the second side gets hotter. The "hot" side is typically attached to a heat sink so that it remains at ambient temperature.

A thermoelectric element provides cooling without a cooling fluid, which has the advantage that potential damage to the patient due to leakage of cooling fluid can be avoided. In addition, thermoelectric elements are available in small sizes, such that the element may be directly placed at the connecting means or cooling element, which reduces losses of cooling capacity due to transport via a cooling fluid or heat conducting leads.

In certain embodiments, the thermoelectric element is electrically connected to a voltage source (e.g. a battery). The voltage source may be comprised in the catheter (e.g., in a handle at the proximal end of the catheter) and electrically connected to the thermoelectric element. Alternatively, the catheter may comprise at least one socket (e.g., at the handle) connected to the thermoelectric element by electrical connections, such that the thermoelectric element may be connected to an external voltage source (i.e., which is not integrated into the catheter) via the at least one socket, optionally further via an adapter.

The electrical connections may be arranged along the inner shaft on the outside of the inner shaft. Alternatively, the electrical connections may be arranged in a wall of the inner shaft or a further shaft of the delivery system, or in a lumen within the inner shaft, the outer shaft or a further shaft. In case the electrical connections are arranged within the lumen of the inner shaft, an additional shaft harboring a guidewire may be provided within the lumen of the inner shaft.

Alternatively, if a connecting means is mounted on an outer shaft, the electrical connections may be arranged in a wall of the outer shaft.

In certain embodiments, the cooling means such as a cooling device, particularly the thermoelectric element thereof, may further be configured to cool a capsule configured to encase the self-expanding structure such as a stent or a heart valve prosthesis, particularly an aortic heart valve prosthesis, in the compressed / crimped state, wherein the capsule is comprised in the outer shaft, and wherein particularly the capsule is retractable relative to the inner shaft. Particularly, a metallic core of the capsule may be cooled by the cooling means, particularly the thermoelectric element. This further increases the contact area to the self-expanding-structure such as a stent, thereby improving heat transfer.

According to the subject-matter of the present invention, particularly cooling of a distal capsule region is advantageous. For instance, a cooled flaring capsule tip could thereby increase efficiency of heat transfer as providing a larger contact area.

In certain embodiments, the cooling means such as a cooling device comprises a cold source and at least one heat conducting lead in thermal contact with the connecting means or the cooling element, wherein the heat conducting lead is configured to conduct heat between the connecting means or the cooling element and the cold source, such that the connecting means or the cooling element is cooled by the cold source via the heat conducting lead.

In particular, this has the advantage that a larger external cold source with a high cooling capacity may be used without the need to provide a cooling fluid in the catheter.

In certain embodiments, the heat conducting lead comprises a thermally isolating coating arranged around the heat conducting lead.

In particular, the heat conducting lead is formed from a material having a sufficient heat conductivity allowing for a suitable cooling of the self-expanding structure. In particular, the heat conducting lead comprises copper or silver, e.g., wherein the heat conducting lead comprises or consists of a silver wire or a copper wire.

The thermal conductivity of a material is a measure of its ability to conduct heat. Heat transfer occurs at a lower rate in materials of low thermal conductivity than in materials of high thermal conductivity. For instance, metals typically have high thermal conductivity and are very efficient at conducting heat, while the opposite is true for insulating materials like Styrofoam. Correspondingly, materials of high thermal conductivity are widely used, e.g., in heat sink applications, and materials of low thermal conductivity are used as thermal insulation. The reciprocal of thermal conductivity is called thermal resistivity.

In particular, the cold source is arranged in a proximal handle of the delivery system, e.g. of a catheter. Alternatively, the heat conducting lead is connected to a socket for connecting an external cold source by a heat conducting lead to the socket, wherein particularly the heat conducting lead comprises a thermally isolating coating or cover. Furthermore, alternatively, the cold source may be positioned in a shaft of the delivery system, wherein particularly, the thermal conduction between the cold source and the connecting means can be interrupted and reestablished, e.g., by a switch.

In certain embodiments, the at least one heat conducting lead is arranged along the inner shaft. In certain embodiments, the at least one heat conducting lead is arranged on the outside of said inner shaft, in a lumen or in a wall of the inner shaft.

In certain embodiments, the delivery system comprises a control device for controlling a temperature of the connecting means and/or the self-expanding structure, wherein the control device is configured to control the cooling means such as a cooling device. Therein, the term "control" particularly incorporates open-loop control and closed-loop control. In particular, the control device is configured to control the cooling performance of the cooling means. To this end, e.g., a voltage applied to a thermoelectric element or a flow rate of a pump circulating cooling fluid through channels of the connecting means or cooling element or thermal conduction connection may be controlled by the control device.

In certain embodiments, the delivery system comprises a first temperature sensor for measuring a temperature of the self-expanding structure, wherein the first temperature sensor is configured to transmit a first measured temperature value representing the current temperature of the self-expanding structure to the control device, wherein the control device is configured to control the cooling means such as a cooling device using the first measured temperature value as an input, wherein particularly the first temperature sensor is in direct thermal contact with the connecting means.

In particular, if the first measured temperature value is above the desired temperature, the control device is configured to increase the cooling performance of the cooling means, and if the first measured temperature value is below the desired temperature, the control device is configured to decrease the cooling performance of the cooling device.

Directly measuring the temperature of the connecting means or self-expanding structure advantageously improves the accuracy of the temperature control, such that the actual temperature of the self-expanding structure is closer to the desired temperature.

In certain embodiments, the delivery system further comprises a second temperature sensor for measuring a surrounding temperature, particularly the body temperature of the bloodstream, wherein the second temperature sensor is configured to transmit a second measured temperature value representing the current surrounding temperature to the control device, wherein the control device is configured to control the cooling means such as a cooling device using the first measured temperature value and the second measured temperature value as an input.

Using the temperature of the local environment measured by the second temperature sensor as an additional parameter for cooling control further improves the accuracy of the obtained self-expanding structure temperature.
In certain embodiments, the second temperature sensor is arranged at a tip section at a distal end of the delivery system.

Further with the context of the invention, an additional temperature sensor upstream from the self-expanding structure, e.g. on an outer shaft proximally from a capsule, may be used to ensure that the blood temperature does not decrease too much during cooling in order to further improve safety for a patient.

In certain embodiments, the delivery system comprises a recapture mechanism configured to initiate recapture of the self-expanding structure between the inner shaft and the outer shaft, wherein the control device is configured to automatically activate the cooling means when the recapture mechanism is activated.
Alternatively, the cooling means may be switched on manually, e.g. by a switch positioned at the handle of the delivery system.

In particular, actuating the recapture mechanism triggers a relative movement between the outer shaft and the inner shaft and/or between the capsule and the inner shaft of the catheter, by which the self-expanding structure is reinserted into the space between the inner shaft and the outer shaft and/or capsule.

For actuating the recapture mechanism an automatic and/or manual actuation is feasible. In certain embodiments, an operator may be free to choose among automatic and/or manual actuation of the delivery system's handle.
In certain embodiments, the delivery system further comprises a signaling device configured to generate a signal when the self-expanding structure or the connecting means has reached the desired temperature.

In particular, the signaling device may be configured to generate an optical, acoustic or haptic signal.

In certain embodiments, the control device is further configured to determine and/or store a number of recapture events that have occurred, wherein the signaling device is configured to indicate / display the number of recapture events that have occurred *inter alia* as a safety measure.

The above described determination and storage of a number of recapture events may also be useful for a delivery system without a cooling mechanism. Such a mechanism can further be combined with a mechanism which blocks the release of a self-expanding structure after a maximally allowed number of recapturing events occured. That being said, the delivery system may anyway comprise a further mechanism to override this block. The blocking mechanism can be, e.g., located in the handle. The counting mechanism can be a mechanical (clockwork-like) or may use sensors. In another embodiment, instead of blocking, a warning signal may be applied.

In certain embodiments, the control device is further configured to determine and/or store a maximum allowable number of recapture events, wherein the signaling device is configured to indicate the maximum allowable number of recapture events.

In certain embodiments, the control device is further configured to determine and/or store an allowable number of remaining recapture events, wherein the signaling device is configured to indicate the allowable number of remaining recapture events.

In certain embodiments, the delivery system comprises a blocking mechanism configured to prevent recapture of the self-expanding structure between the inner shaft and the outer shaft, wherein the control device is configured to deactivate the blocking mechanism when the self-expanding structure or the connecting means has reached the desired temperature.

In certain embodiments, the blocking mechanism is configured to be manually deactivated, e.g. by a switch at the catheter handle.

In certain embodiments, the signaling device is configured to generate a warning signal when the blocking mechanism is manually deactivated.

In particular, the blocking mechanism prevents relative movement between the outer shaft and the inner shaft or between the capsule and the inner shaft of the catheter.

A further aspect of the invention relates to an assembly of a delivery system according to the invention and a self-expanding structure such as a stent or a heart valve prosthesis.

Wherever alternatives for single separable features are laid out herein as "embodiments", it is to be understood that for a person skilled in the art while having regard to his/her common general knowledge such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

It shall be noted that the disclosed figures predominantly serve the purpose of illustrating various exemplary embodiments of the invention without being bound to scales, dimensions, relations or the like. Various modifications and/or arrangements and/or implementations of the illustrated embodiments may be conceived by a skilled person with regard to his/her common general knowledge. Thus, each illustrated component / feature shall predominantly illustrate the basic arrangements, functions and interplay thereof in accordance with the invention.
- Fig. 1: shows a schematic partial sectional view of an exemplary delivery system such as a catheter according to a first embodiment of the invention with an exemplary stent as self-expanding structure in the compressed / crimped state and a conduction-based cooling mechanism in accordance with the invention;
- Fig. 2: shows a schematic sectional view of a part of the delivery system such as a catheter according to Fig. 1 with the exemplary stent in a partially deployed state;
- Fig. 3: shows a sectional view of a part of the delivery system such as a catheter according to Fig. 1, but while illustrating an embodiment of the connecting means (such as a prosthesis connector) having tiltable cooling / connecting surfaces for fixing and adaptation thereof to a self-expanding implant (e.g. via its eyelets or tabs) that may move in the region of fixation during deployment / recapture / re-sheathing; in this embodiment each of the tiltable cooling / connecting surfaces is cooled individually by a separate conduit for cooling;
- Fig. 4: shows a sectional view of an exemplary delivery system such as a catheter according to a second embodiment of the invention with a cooling mechanism based on a circulating cooling fluid;
- Fig. 5: shows a sectional view of an exemplary delivery system such as a catheter according to a third embodiment of the invention with a cooling mechanism based on a thermoelectric element;
- Fig. 6: shows a sectional view of an exemplary delivery system such as a catheter according to a fourth embodiment of the invention with a conduction-based cooling mechanism and an additional cooling element in contact with the connecting means such as a prosthesis connector;
- Fig. 7: shows a sectional view of an exemplary delivery system such as a catheter according to a fifth embodiment of the invention with a cooling mechanism based on a circulating cooling fluid and an additional cooling element in contact with the connecting means such as a prosthesis connector;
- Fig. 8: shows a sectional view of an exemplary delivery system such as a catheter according to a sixth embodiment of the invention with a cooling mechanism based on a thermoelectric element and an additional cooling element in contact with the connecting means such as a prosthesis connector.

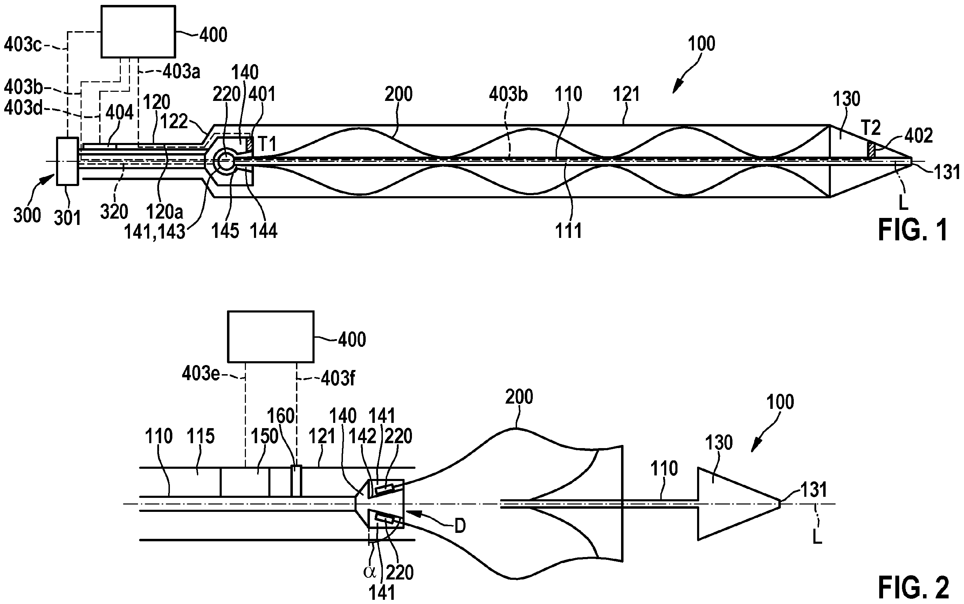

Fig. 1 shows a sectional view of a distal portion of a delivery system such as a catheter 100 for delivery and deployment of a self-expanding structure such as a stent 200 in a human or animal body. The catheter 100 extends along a longitudinal axis L between a proximal end (not shown) and a distal end formed by the tip 131. The distal end of the catheter is configured to be inserted into a patient vessel or hollow organ, particularly a blood vessel (further locations may be the bile ducts, urinary tract, etc.), and the proximal end of the catheter 100 particularly comprises a handle with an actuating mechanism to *inter alia* guide and optionally steer the catheter 100 through the patient vessel or hollow organ.

The catheter 100 comprises an inner shaft 110 harboring a guidewire lumen 111 and extending along the longitudinal axis L towards (and particularly through) a tip section 130 (also termed nosecone) comprising the tip 131 at the distal end. Furthermore, the catheter 100 comprises an outer shaft 120 extending along the longitudinal axis L parallel to the inner shaft 110, wherein the outer shaft 120 is arranged, particularly coaxially, around the inner shaft 110, such that a lumen 115 is formed between the inner shaft 110 and the outer shaft 120 (see Fig. 2). In the example of a catheter as delivery system as shown in Fig. 1, the outer shaft 120 comprises a main portion 120a and a retractable capsule 121 arranged distally of the main portion 120a and particularly connected to the main portion 120a by a tapered connection 122. Typically, a capsule is fixed to an outer shaft and the outer shaft and the capsule are retracted together as one structure. The capsule 121 is typically formed from a metallic material core (e.g. laser-cut metal tube, braided tube, or e.g. wired tube) optionally having an outer polymer jacket and/or an inner polymer liner, and particularly has a larger diameter compared to the main portion 120a. To mount, deploy and recapture a self-expanding structure such as a stent 200, the capsule 121 is movable along the longitudinal axis L relative to the inner shaft 110. Alternatively, if there is no capsule 121, the entire outer shaft 120 may be movable relative to the inner shaft 110 for mounting, deployment and recapture of the self-expanding structure such as a stent 200.

In the given example of Fig. 1, to mount the stent 200 to the catheter 100, the stent 200 is particularly crimped / compressed onto the inner shaft 110 with the capsule 121 retracted to accommodate the stent 200, and the capsule 121 is subsequently advanced in the distal direction towards the tip section 130 to cover the stent 200 and prevent premature expansion of the stent 200. Crimping / compressing is particularly done at low temperature facilitating plastic deformation of the stent with low mechanical stress. The stent 200 may be also fixed to a connecting means 140 through a loading procedure, e.g. with a cone shaped loading tool.

For additional fixation of the stent 200, particularly in the partially deployed state, the catheter 100 comprises a connecting means such as a prosthesis connector 140 and/or a connector arm and/or a connector feeler, which is particularly fixed to the inner shaft 110 of the catheter 100. In the examples shown in Figs. 1 and 2, the prosthesis connector 140 as exemplary connecting means is placed quite adjacent to the proximal end of the capsule 121, i.e. quite nearby the tapered connection 122 between the capsule 121 and the main portion 120a of the outer shaft 120. Fig. 1 shows a side view of the prosthesis connector 140 and Fig. 2 shows a sectional view of the prosthesis connector 140 as exemplary connecting means in accordance with the invention, wherein the view of Fig. 2 is rotated around the longitudinal axis L.

As displayed in Figs. 1 and 2, the exemplary prosthesis connector 140 comprises at least one recess 141, whereas two or three, or more recesses are preferred (i.e. a plurality of recesses)configured to be engaged by respective connecting elements 220 (e.g. eyelets or tabs) of the stent 200 disposed at the proximal end of the stent 200. For example, the connecting elements 220 may be eyelets (i.e., round or oval shaped loop like structures having a central opening) or tabs (i.e., elongated, round, oval, rectangular, T-shaped or other structures). In case the self-expanding structure (such as a stent, an implant, a heart valve prosthesis, particularly an aortic heart valve prosthesis) itself has eyelets, the connecting means such as a prosthesis connector 140 of the catheter may have one or more protrusions for connection.

In the given example, the recesses 141 shown in Figs. 1 and 2 have a shape resembling a keyhole when viewed from the side (see Fig. 1) with a proximal circular segment 143 and a distal elongated segment 144 optionally tapering towards the outside, wherein the circular segment 143 is connected to the elongated segment 144 at a constriction 145. As shown in Fig. 1, a connecting element 220 in form of an eyelet or tab of an appropriate size can be inserted into the circular segment 143. In case the eyelet is larger than the constriction 145 between the circular segment 143 and the elongated segment 144 of the recess 141, the stent 200 is thereby axially fixed to the connector 140.

In an embodiment, an eyelet could be filled with a material having properties that allow for an improved heat transfer. Following this, in an embodiment a marker element (e.g. gold, tantalum, and the like) could be applied to the eyelet for improving heat transfer and visibility during a suitable imaging techniques such as fluoroscopy.

As an alternative to an eyelet as connecting element, a respective tab may achieves a larger contact area for heat transfer. Optionally, an eyelet, however, may be filled with a material having properties that allow for an improved heat transfer. Optionally, an optical marker element (e.g. gold, tantalum etc.) may be used for improving heat transfer and visibility during imaging such as fluoroscopy.

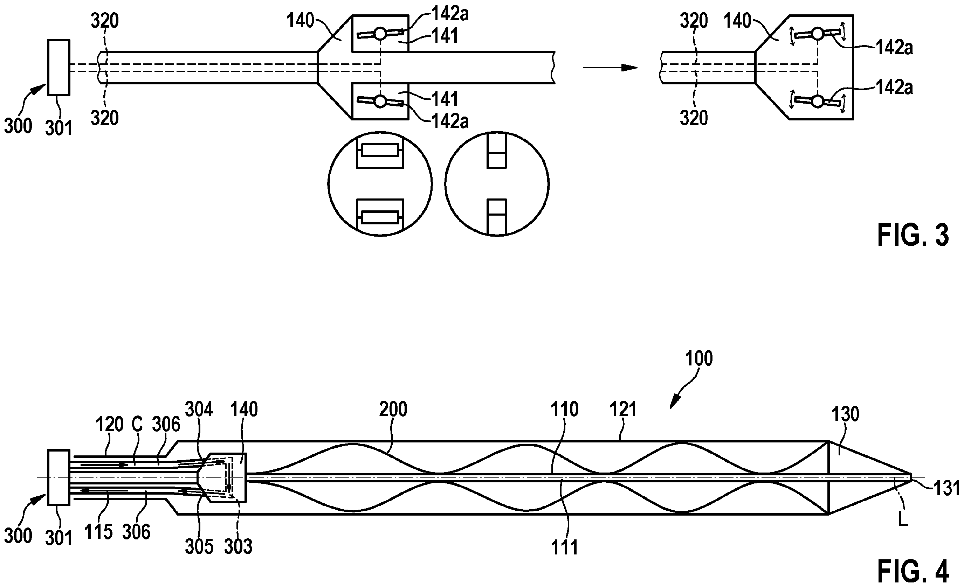

Fig. 2 shows two of the recesses 141 of the connector 140 as exemplary connecting means in cross-section. The recesses 141 comprise contacting surfaces 142 at the bottom of the respective recesses 141 (being optionally configured as pivotally mounted recesses that are tiltable; cf. Fig. 3) which are configured to contact the connecting elements 220 when the connecting elements 220 engage the recesses 141 (especially when self-expanding structure such as an implant is in partially expanded configuration), serving as a heat bridge to cool the stent 200 as exemplary self-expanding structure as explained below. In this illustrated embodiment, the contacting surfaces 142 are inclined by an inclination angle α in respect of the longitudinal axis L. This results in an improved contact between the connecting elements 220 and the contacting surfaces 142 in the partially deployed state of the stent 200 as displayed in Fig. 2. The contacting surfaces 142 and/or the connecting elements 220 may also be covered by a soft material exhibiting good heat conductivity to improve contact between the connecting surfaces 142 and the connecting elements 220.

Fig. 3 shows two of the recesses 141 of the connector 140 in cross-section. Here, the recesses 141 comprise pivotable and/or tiltable contacting surfaces 142a at the bottom of the respective recesses 141 which are configured to movably contact the connecting elements 220 (not shown) when the connecting elements 220 engage the recesses 141, serving as a movable heat bridge to cool the stent 200 during, e.g., an entire resheathing event. Thus, the contacting surfaces 142a are pivotable and/or tiltable by an inclination angle α in relation to the movements of the stent 200 during a resheathing. This results in an improved contact between the connecting elements 220 and the pivotable / tiltable contacting surfaces 142a throughout the partially deployed state of the stent 200. Thereby, the said contacting surfaces 142a and/or the connecting elements 220 (not shown) may also be covered by a soft material exhibiting good heat conductivity to improve contact between the pivotable and/or tiltable contacting surfaces 142a and the connecting elements 220 (not shown).

In view of the embodiment of Fig. 3, and especially when a self-expanding structure is in a partially expanded configuration, the contacting surfaces 142a (i.e. connecting element 220 receptacles) with thermal contact surfaces that are pivotably and/or tiltable mounted on a connecting means such as a prosthesis connector 140 allow for a maximized contact area between the connector means and the self-expanding structure over a large range of expansion states of the self-expanding structure, and thus thereby reduce strains.

Such a configuration of pivotable and/or tiltable and/or movable contacting surfaces 142a for connecting elements 220 of a self-expanding structure may be also useful for delivery systems without a cooling mechanism.

Thus, in one embodiment, the present disclosure provides for pivotable and/or tiltable and/or movable contacting surfaces 142a, e.g. of a connecting means such as a prosthesis connector, for a releasable engagement with one or more connecting elements 220 of a self-expanding structure with the context of the cooling mechanisms of the present invention or with the context of a delivery system without a cooling mechanism.

Returning now to Fig. 1, the catheter 100 further comprises a cooling means such as a cooling device 300 which in case of the exemplary embodiment shown in Fig. 1 incorporates a cold source 301 and a heat conducting lead 320 connecting the cold source 301 to the connecting means such as a prosthesis connector 140 to cool the prosthesis connector 140.

The cold source 301 may be configured to generate cold by any cooling mechanism known from the state of the art, e.g., vapor compression cooling, vapor absorption cooling, adsorption cooling, diffusion-absorption cooling, steam jet cooling, the Joule-Thomson effect, pulse tube refrigeration, the thermoelectric effect, magnetic cooling or evaporation cooling.

Furthermore, the cold source 301 may be positioned within the delivery system such as a catheter 100 (particularly in a handle at the proximal end of the catheter) or as a separate unit.

One or several heat conducting leads 320 may be provided to connect the cold source 301 to the connecting means such as a prosthesis connector 140. In particular, the heat conducting leads 320 may comprise or consist of a wire from a metal exhibiting good heat conductivity, e.g., silver or copper. To prevent loss of cooling performance, the heat conducting leads 320 may comprise a thermally isolating coating or cover. In the example depicted in Fig. 1, the heat conducting lead 320 is positioned in the lumen 115 between the inner shaft 110 and the outer shaft 120, but of course placement in other locations is also possible. For instance, an attachment to the inner shaft may be beneficial to prevent the conducting lead 320 from moving around and so to block shafts and/or break. An attached conducting lead 320 may even reduce friction between shafts by reducing contact surface between shafts.

Fig. 1 further shows a control device 400 for controlling the cooling device 300, e.g., by open loop control or closed loop control. The control device 400 is connected to a first temperature sensor 401 placed in direct contact with the prosthesis connector 140 via a first data connection 403a, connected to a second temperature sensor 402 at the distal tip section 130 of the catheter 100 via a second data connection 403b and connected to the cold source 301 of the cooling device 300 as cooling means via a third data connection 403c. The first temperature sensor 401 is configured to measure a first temperature T1 of the prosthesis connector 140 representing the approximate temperature of the stent 200 connected to the prosthesis connector 140, and the second temperature sensor 402 is configured to measure a second temperature T2 representing the temperature of the surrounding environment, i.e., typically the bloodstream of the patient. The first temperature sensor 401 and the second temperature sensor 402 are configured to transmit the measured temperature to the control device 400 via the respective data connections 403a, 403b, and the control device 400 is configured to use the transmitted temperature values to determine how the cooling device 300 as cooling means (in this example the cold source 301) needs to be controlled to obtain the desired temperature of the stent 200. For example, if the measured first temperature T1 is above the desired temperature, the control device 400 may transmit a signal to the cooling device 300, particularly to the cold source 301, and so to increase the cooling performance of the cooling device 300 as cooling means. The second temperature T2 of the surrounding environment may be included in the control mechanism of the control device 400 to increase the accuracy of temperature control; e.g. a more intense cooling for higher blood temperatures.

In addition, the catheter 100 may comprise an optical, acoustic or haptic signaling device 404 (see a schematic drawing thereof in Fig. 1), particularly arranged at the handle at the proximal end of the catheter 100 (not shown). The signaling device 404 is particularly configured to generate an acoustic, optical or haptic signal to the physician operating the catheter 100 when the desired temperature of the stent 200 has been reached. In particular, the control device 400 is connected to the signaling device 404 by a fourth data connection 403d, such that an electric signal may be transmitted from the control device 400 to the signaling device 404 if the measured first temperature T1 received by the control device 400 from the first temperature sensor 401 is sufficiently close to the desired temperature. This may be realized while the heat flow is measured to detect when equilibrium temperature is approached. The optical, acoustic or haptic signal particularly indicates when the physician may safely initiate recapture of the stent 200 (i.e. a resheathing).

As depicted in Fig. 2, the catheter 100 as exemplary delivery system may further comprise a recapture mechanism 150 (only schematically shown, typically located as an actuator in a handle portion of the catheter) to initiate recapture of the stent 200 between the inner shaft 110 and the outer shaft 120 and/or a capsule 121. The recapture mechanism 150 is particularly a mechanical mechanism that triggers a relative movement between the capsule 121 and/or outer shaft 120 and the inner shaft 110. In particular, the recapture mechanism 150 may be connected to the control device 400 by a fifth data connection 403e, wherein when the recapture mechanism 150 is initiated by the physician, the recapture mechanism 150 is configured to transmit an electric signal to the control device 400, and the control device 400 is configured to activate the cooling device 300 upon receipt of said signal. In particular, at the same time, a blocking mechanism 160 may be activated by the control device 400 by transmitting an electric signal from the control device 400 to the blocking mechanism 160 via a sixth data connection 403f. The blocking mechanism 160 particularly prevents a relative movement between the capsule 121 and/or outer shaft 120 and the inner shaft 110, so that no recapture is possible. In particular, when the control device 400 receives temperature data indicating that the stent 200 has reached the desired temperature, the control device 400 deactivates the blocking mechanism 160 (the blocking mechanism may be also used for blocking after a maximum number of re-sheathing events occurred, cf. above). Subsequently, the recapture mechanism 150 may be manually or automatically activated to initiate recapture of the stent 200. With this context, a signal for the status of the blocking mechanism may be applied such as an optical, acoustic or haptic signal or the like.

A blocking mechanism may be also useful in case a maximum number of counted resheathing events occurred (see above).

Fig. 4 shows a second embodiment of the catheter 100 as exemplary delivery system according to the invention, where the cooling means is a cooling device 300 comprising a cold source 301 configured to cool a cooling fluid C, e.g., isotonic NaCl solution, wherein the cold source 301 is connected to a channel 303 of the connector 140 by conduits 306, such that the cold cooling fluid C may circulate through the channel 303 as indicated by the arrows in Fig. 4, thereby cooling the connector 140 as connecting means.

The channel 303 of the connector 140 extends between a first opening 304 and a second opening 305, the openings 304, 305 being exemplarily oriented towards the lumen between the inner shaft 110 and the capsule 121 (optionally other orientations are possible), wherein the openings 304, 305 are positioned on the proximal side of the connector 140. The openings 304, 305 are each connected to a respective conduit 306 leading to the cold source 301.

The cold source 301 may use any kind of mechanism for generating cold, e.g., those described above for the embodiment of Fig. 1. Furthermore, the cold source 301 may be disposed within the catheter 100 (e.g., in the handle) or the cold source 301 may be a separate unit arranged outside of the catheter 100. In particular, the cold source 301 comprises a pump to generate a current of the cooling fluid C between the cold source 301 and the channel 303. Alternatively, an external pump may be used for this purpose.

The conduits 306 may be a tubing that is particularly arranged in the lumen 115 of the catheter 100 between the inner shaft 110 and the outer shaft 120. Alternatively, the conduit 306 may be a lumen of the catheter 100 itself, e.g., the lumen 115 between the inner shaft 110 and the outer shaft 120 may be used as conduits 306.
In a similar manner as described above for the embodiment shown in Fig. 1, the catheter 100 may comprise a control device 400 to control the cooling device 300. In case of the embodiment of Fig. 4, the control device 400 is particularly connected to the cold source 301 to control the cooling performance of the cold source 301 using the measured temperatures of the temperature sensors 401, 402 as input values (see Fig. 1).

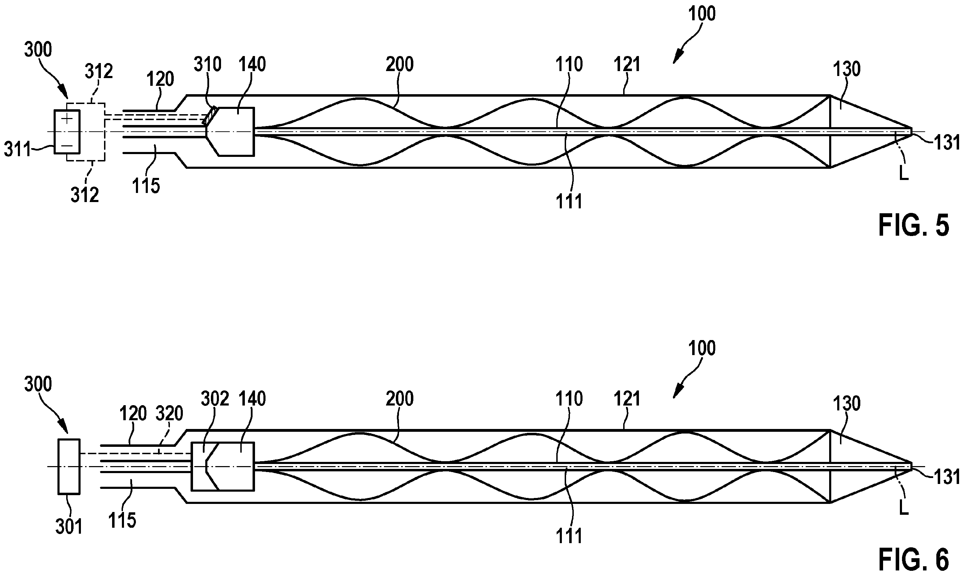

Fig. 5 shows a third embodiment of the catheter 100 as exemplary delivery system according to the invention, in which the cooling device 300 comprises a thermoelectric element 310 in direct thermal contact with the connector 140 as connecting means, wherein the thermoelectric element 310 is connected to a voltage source 311 (such as a battery or grid) by electrical connections 312, resulting in a direct current through the thermoelectric element 310, so that the connector 140 is cooled by the thermoelectric element 310.

The voltage source 311 may be arranged within the catheter 100 (e.g. in the handle at the proximal end of the catheter) or may be provided as a separate, external unit.

In the example shown in Fig. 5, the electrical connections 312 are positioned in the lumen 115 between the inner shaft 110 and the outer shaft 120. However, any other suitable lumen of the catheter 100 may also be used to this end.

The catheter 100 shown in Fig. 5 may also comprise a control device 400 to control the thermoelectric element 310. In this case, the control device 400 may be adapted to control the cooling performance of the thermoelectric element 310 using the measured temperatures of the one or more temperature sensors, such as 401, 402, as input values as described above (see Fig. 1). To this end, the control device 400 is particularly connected to the voltage source 311 by a third data connection 403c (not shown) to control the voltage applied by the voltage source 311 and hence the direct current through the thermoelectric element 310.

A fourth embodiment of the catheter 100 according to the invention is illustrated in Fig. 6. This embodiment is identical to the first embodiment shown in Fig. 1, except for an additional cooling element 302 which is in direct thermal contact with the connector 140 as connecting means, wherein the cooling element 302 is connected to the cold source 301 by a heat conducting lead 320.

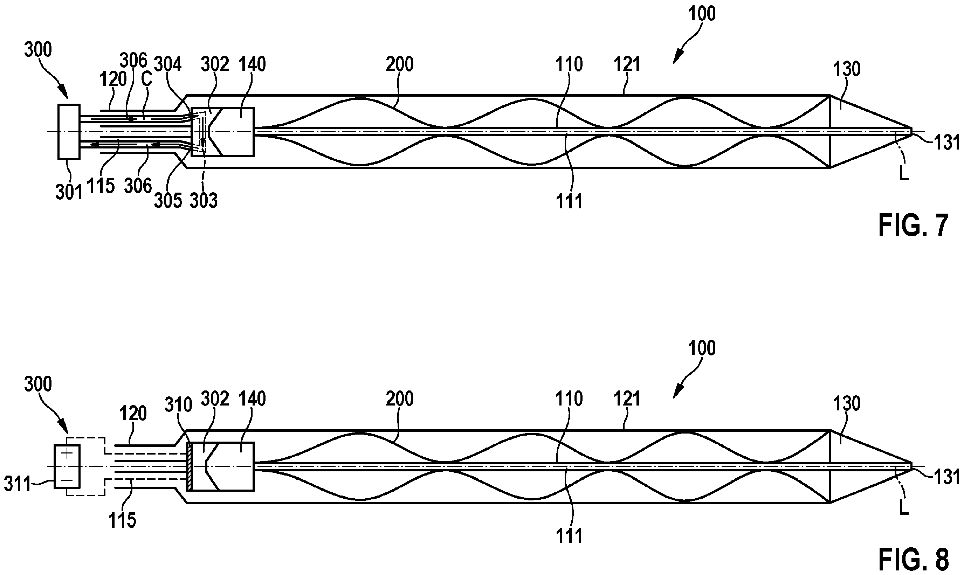

Furthermore, Fig. 7 shows a fifth embodiment of the catheter 100 as exemplary delivery system according to the invention which is identical to the second embodiment shown in Fig. 4, except that a cooling element 302 in direct thermal contact with the connector 140 is provided. The cooling element 302 comprises a channel 303 extending between a first opening 304 and a second opening 305, wherein the first opening 304 and the second opening 305 are each connected to a conduit 306 which is in turn connected to the cold source 301, such that a cooling fluid C may be circulated through the channel 303 to cool the cooling element 302, which in turn cools the connector 140 as exemplary connecting means by heat conduction.

Fig. 8 depicts a sixth embodiment of the exemplary catheter 100 according to the invention identical to the third embodiment shown in Fig. 5, but with the difference that the catheter 100 comprises a cooling element 302 in direct thermal contact with the connector 140 as exemplary connecting means, wherein the thermoelectric element 310 of the cooling device 300 is attached to the cooling element 302, such that the cooling element 302 is cooled by the thermoelectric element 310 and the connector 140 is in turn cooled by the cooling element 302.

### List of reference numerals

- 100: Catheter
- 110: Inner shaft
- 111: Guidewire lumen
- 115: Lumen
- 120: Outer shaft
- 120a: Main portion
- 121: Capsule
- 122: Tapered connection
- 130: Tip section
- 131: Tip
- 140: Connector
- 141: Recess
- 142: Contacting surface
- 142a: Pivotable / tiltable / movable contacting surface
- 143: Circular segment
- 144: Elongated segment
- 145: Constriction
- 150: Recapture mechanism
- 160: Blocking mechanism
- 200: Stent
- 220: Connecting element
- 300: Cooling device
- 301: Cold source
- 302: Cooling element
- 303: Channel
- 304: First opening
- 305: Second opening
- 306: Conduit
- 310: Thermoelectric element
- 311: Voltage source
- 312: Electrical connection
- 320: Heat conducting lead
- 400: Control device
- 401: First temperature sensor
- 402: Second temperature sensor
- 403a, 403b, 403c, 403d, 403e, 403f: Data connection
- 404: Signaling device
- α: Inclination angle
- C: Cooling fluid
- D: Distal end
- L: Longitudinal axis
- T1: First measured temperature value
- T2: Second measured temperature value

In view of all the foregoing disclosure, the present invention further provides for the below consecutively numbered embodiments:
1. A delivery system (100) adapted for delivery and deployment of a self-expanding structure (200) in a human or animal body, comprising
   - an inner shaft (110) extending along a longitudinal axis (L),
   - an outer shaft (120) arranged around the inner shaft (110) and optionally comprising a capsule (121) at a distal end of the outer shaft, wherein the delivery system (100) is configured to receive a self-expanding structure (200) between the inner shaft (110) and the outer shaft (120) and/or the capsule (121) in a compressed or crimped state, and
   - a cooling means (300) for cooling the self-expanding structure (200) to a desired temperature, thereby facilitating recapture of the self-expanding structure (200) between the inner shaft (110) and the outer shaft (120) and/or the capsule (121),
   **characterized in that**
   the delivery system (100) comprises a connecting means (140) optionally comprising a connecting arm and/or a connecting feeler for releasably fixing the self-expanding structure (200) to the delivery system (100), wherein the connecting means (140) and/or connecting arm and/or connecting feeler is coolable by the cooling means (300).
   Throughout the description portion and in view of the above embodiment 1, the term "connecting means" shall be understood as a mechanical means being suitable for releasably fixing / holding a self-expanding structure. Such a connecting means may be exemplarily selected from a prosthesis connector, a connecting arm, a connecting feeler, a connecting tether, or combinations thereof. Thus, with the context of the invention, a connecting means may be reflected by a prosthesis connector having additional one or more connecting arms and/or connecting feeler and/or connecting tether or the connecting means may be reflected by one or more connecting arms and/or connecting feeler and/or connecting tether without being arranged on a prosthesis connector.
2. The delivery system (100) according to embodiment 1, **characterized in that** the connecting means (140) comprises at least one recess (141) and/or protrusion configured to be releasably engaged by a connecting element (220), particularly a holding means such as an eyelet or a tab, of the self-expanding structure (200) to releasably fix the self-expanding structure (200) to the delivery system (100), wherein the recess (141) and/or protrusion comprises a contacting surface (142) and/or a pivotable and/or a tiltable contacting surface (142a) configured to contact the connecting element (220), particularly the holding means such as the eyelet or the tab, for conducting heat between the connecting element (220) and the contacting surface (142) and/or the pivotable and/or the tiltable contacting surface (142a).
   Throughout the specification and in view of the above embodiment 2, the expressions "contacting surface (142)" and "pivotable and/or a tiltable contacting surface (142a)" shall be understood as being different embodiments in certain instances with the context of the invention. That is, a contacting surface (142) as such, basically, reflects some sort of a mechanically fixed contacting surface, whereas a pivotable and/or a tiltable contacting surface (142a) reflects some sort of a non-fixed, but movable contacting surface that is movably / pivotally mounted, e.g., on a connecting means, and thus allows for a pivotable and/or tiltable movement. In certain instances, however, the more general expression contacting surface (142) may be understood as including both a fixed and a non-fixed contacting surface.
3. The delivery system (100) according to embodiment 2, **characterized in that** the connecting means (140) comprises a distal end (D), wherein the recess (141) and/or protrusion extends towards the distal end (D), such that the self-expanding structure (200) extends from the distal end (D) when the connecting element (220), particularly the holding means such as the eyelet or the tab, engages the recess (141) and/or protrusion, wherein the contacting surface (142) is inclined toward said distal end (D) by an inclination angle (α) with respect to the longitudinal axis (L), wherein particularly the inclination angle is 5° to 60°, wherein more particularly the inclination angle is 15° to 45°.
   Throughout the specification and in view of the above embodiment 3, the expression "inclination angle (α)" may be read on both types of contacting surfaces (142, 142a). That is, a mechanically fixed type of contacting surface (142) will also have a fixed inclination angle (α), e.g., within the above described ranges of embodiment 3. In contrast, a mechanically non-fixed type of contacting surface (142a) will have some sort of a varying inclination angle (α) depending on the exact embodiment.
4. The delivery system (100) according to embodiment 2 or 3, **characterized in that** the contacting surface (142) and/or the pivotable and/or the tiltable contacting surface (142a) or the connecting element (220) is at least partially covered with a soft heat-conducting material, particularly with a polymer filled with metal powder.
5. The delivery system (100) according to one of the preceding embodiments, **characterized in that** the delivery system (100) comprises a cooling element (302) which is in thermal contact with the connecting means (140), wherein the cooling means (300) is configured to cool the cooling element (302).
6. The delivery system (100) according to one of the preceding embodiments, **characterized in that** the cooling means (300) comprises a cold source (301) configured to cool a cooling fluid (C) and to provide the cooling fluid (C) at the connecting means (140) and/or the cooling element (302), such that the connecting means (140) and/or the cooling element (302) is coolable by the cooling fluid (C).
7. The delivery system (100) according to embodiment 6, **characterized in that** the connecting means (140) or the cooling element (302) comprises at least one channel (303) for conducting the cooling fluid (C), wherein the channel (303) comprises a first opening (304) and a second opening (305), and wherein the first opening (304) and the second opening (305) are connected to the cold source (301), such that the cooling fluid (C) is able to flow from the cold source (301) into the first opening (304), through the channel (303), and through the second opening (305), particularly back to the cold source (301), wherein particularly the cooling means (300) comprises at least one conduit (306) for conducting the cooling fluid (C) between the cold source (301) and the channel (303), wherein more particularly the conduit (306) is either formed by a lumen (115) of the delivery system (100), wherein even more particularly the lumen (115) is arranged between the inner shaft (110) and the outer shaft (120) and/or the capsule (121) of the delivery system (100) or the conduit is formed by a separate tubing.
8. The delivery system (100) according to one of the preceding embodiments, **characterized in that** the cooling means (300) comprises a thermoelectric element (310), particularly a Peltier element, in thermal contact with the connecting means (140) or the cooling element (302), wherein the thermoelectric element (310) is configured to cool the connecting means (140) or the cooling element (302).
9. The delivery system (100) according to one of the preceding embodiments, **characterized in that** the cooling means (300) comprises a cold source (301) and at least one heat conducting lead (320) in thermal contact with the connecting means (140) or the cooling element (302), wherein the heat conducting lead (320) is configured to conduct heat between the connecting means (140) or the cooling element (302) and the cold source (301), such that the connecting means (140) or the cooling element (302) is cooled by the cold source (301) via the heat conducting lead (320), wherein particularly the heat conducting lead (320) comprises a thermally isolating coating or cover arranged around the heat conducting lead (320).
10. The delivery system (100) according to one of the preceding embodiments, **characterized in that** the delivery system (100) comprises a control device (400) for controlling a temperature of the connecting means (140) and/or the self-expanding structure (200), wherein the control device (400) is configured to control the cooling means (300).
11. The delivery system (100) according to embodiment 10, **characterized in that** the delivery system (100) comprises a first temperature sensor (401) for measuring a temperature of the connecting means (140), wherein the first temperature sensor (401) is configured to transmit a first measured temperature value (T1) to the control device (400), and wherein the delivery system (100) further comprises an additional sensor for measuring a heat flow and which is configured to transmit a first measured heat flow (HF1) to the control device (400), and wherein the control device (400) is configured to control the cooling means (300) using the first measured temperature value (T1) and the first measured heat flow (HF1) as an input, wherein particularly the first temperature sensor (401) is in direct thermal contact with the connecting means (140).
12. The delivery system (100) according to embodiment 11, **characterized in that** the delivery system (100) further comprises a second temperature sensor (402) for measuring a surrounding temperature, wherein the second temperature sensor (402) is configured to transmit a second measured temperature value (T2) to the control device (400), wherein the control device (400) is configured to control the cooling device (300) using the first measured temperature value (T1) and the second measured temperature value (T2) as an input and optionally using the first measured heat flow (HF1) as a further input, wherein particularly the second temperature sensor (402) is arranged at a tip section (130) at a distal end of the delivery system (100).
13. The delivery system (100) according to one of the embodiments 10 to 12, **characterized in that** the delivery system (100) comprises a recapture mechanism (150) configured to initiate recapture of the self-expanding structure (200) between the inner shaft (110) and the outer shaft (120) and/or the capsule (121), wherein the control device (400) is configured to automatically activate the cooling means (300) when the recapture mechanism (150) is activated.
14. The delivery system (100) according to one of the embodiments 10 to 13, **characterized in that** the delivery system (100) further comprises a signaling device (404) configured to generate a signal when the self-expanding structure (200) or the connecting means (140) has reached the desired temperature, wherein particularly the control device (400) is further configured to store a number of recapture events that have occurred, a maximum allowable number of recapture events and/or an allowable number of remaining recapture events, wherein the signaling device (404) is configured to indicate the number of recapture events that have occurred and/or the allowable number of remaining recapture events.
   In view of the above embodiment 14, alternatively, the present invention also provides for a delivery system (100) according to embodiment 14, but without the implementation of a cooling mechanism of the invention.
15. The delivery system (100) according to one of the embodiments 10 to 14, **characterized in that** the delivery system (100) comprises a blocking mechanism (160) configured to prevent recapture of the self-expanding structure (200) between the inner shaft (110) and the outer shaft (120) and/or the capsule (121), wherein the control device (400) is configured to deactivate the blocking mechanism (160) when the self-expanding structure (200) or the connecting means (140) has reached the desired temperature, wherein particularly the blocking mechanism (160) is configured to be manually deactivated, wherein more particularly the signaling device (404) is configured to generate a warning signal when the blocking mechanism (160) is manually deactivated.

In view of the above embodiment 15, alternatively, the present invention also provides for a delivery system (100) according to embodiment 15, but without the implementation of a cooling mechanism of the invention.

## Claims

1. A delivery system (100) adapted for delivery and deployment of a self-expanding structure (200) in a human or animal body, comprising
- an inner shaft (110) extending along a longitudinal axis (L),
- an outer shaft (120) arranged around the inner shaft (110) and optionally comprising a capsule (121) at a distal end of the outer shaft, wherein the delivery system (100) is configured to receive a self-expanding structure (200) between the inner shaft (110) and the outer shaft (120) and/or the capsule (121) in a compressed or crimped state, and
- a cooling means (300) for cooling the self-expanding structure (200) to a desired temperature, thereby facilitating recapture of the self-expanding structure (200) between the inner shaft (110) and the outer shaft (120) and/or the capsule (121),
**characterized in that**
the delivery system (100) comprises a connecting means (140) optionally comprising a connecting arm and/or a connecting feeler for releasably fixing the self-expanding structure (200) to the delivery system (100), wherein the connecting means (140) and/or connecting arm and/or connecting feeler is coolable by the cooling means (300).

2. The delivery system (100) according to claim 1, **characterized in that** the connecting means (140) comprises at least one recess (141) and/or protrusion configured to be releasably engaged by a connecting element (220), particularly a holding means such as an eyelet or a tab, of the self-expanding structure (200) to releasably fix the self-expanding structure (200) to the delivery system (100), wherein the recess (141) and/or protrusion comprises a contacting surface (142) and/or a pivotable and/or a tiltable contacting surface (142a) configured to contact the connecting element (220), particularly the holding means such as the eyelet or the tab, for conducting heat between the connecting element (220) and the contacting surface (142) and/or the pivotable and/or the tiltable contacting surface (142a).

3. The delivery system (100) according to claim 2, **characterized in that** the connecting means (140) comprises a distal end (D), wherein the recess (141) and/or protrusion extends towards the distal end (D), such that the self-expanding structure (200) extends from the distal end (D) when the connecting element (220), particularly the holding means such as the eyelet or the tab, engages the recess (141) and/or protrusion, wherein the contacting surface (142) is inclined toward said distal end (D) by an inclination angle (α) with respect to the longitudinal axis (L), wherein particularly the inclination angle is 5° to 60°, wherein more particularly the inclination angle is 15° to 45°.

4. The delivery system (100) according to claim 2 or 3, **characterized in that** the contacting surface (142) and/or the pivotable and/or the tiltable contacting surface (142a) or the connecting element (220) is at least partially covered with a soft heat-conducting material, particularly with a polymer filled with metal powder.

5. The delivery system (100) according to one of the preceding claims, **characterized in that** the delivery system (100) comprises a cooling element (302) which is in thermal contact with the connecting means (140), wherein the cooling means (300) is configured to cool the cooling element (302).

6. The delivery system (100) according to one of the preceding claims, **characterized in that** the cooling means (300) comprises a cold source (301) configured to cool a cooling fluid (C) and to provide the cooling fluid (C) at the connecting means (140) and/or the cooling element (302), such that the connecting means (140) and/or the cooling element (302) is coolable by the cooling fluid (C).

7. The delivery system (100) according to claim 6, **characterized in that** the connecting means (140) or the cooling element (302) comprises at least one channel (303) for conducting the cooling fluid (C), wherein the channel (303) comprises a first opening (304) and a second opening (305), and wherein the first opening (304) and the second opening (305) are connected to the cold source (301), such that the cooling fluid (C) is able to flow from the cold source (301) into the first opening (304), through the channel (303), and through the second opening (305), particularly back to the cold source (301), wherein particularly the cooling means (300) comprises at least one conduit (306) for conducting the cooling fluid (C) between the cold source (301) and the channel (303), wherein more particularly the conduit (306) is either formed by a lumen (115) of the delivery system (100), wherein even more particularly the lumen (115) is arranged between the inner shaft (110) and the outer shaft (120) and/or the capsule (121) of the delivery system (100) or the conduit is formed by a separate tubing.

8. The delivery system (100) according to one of the preceding claims, **characterized in that** the cooling means (300) comprises a thermoelectric element (310), particularly a Peltier element, in thermal contact with the connecting means (140) or the cooling element (302), wherein the thermoelectric element (310) is configured to cool the connecting means (140) or the cooling element (302).

9. The delivery system (100) according to one of the preceding claims, **characterized in that** the cooling means (300) comprises a cold source (301) and at least one heat conducting lead (320) in thermal contact with the connecting means (140) or the cooling element (302), wherein the heat conducting lead (320) is configured to conduct heat between the connecting means (140) or the cooling element (302) and the cold source (301), such that the connecting means (140) or the cooling element (302) is cooled by the cold source (301) via the heat conducting lead (320), wherein particularly the heat conducting lead (320) comprises a thermally isolating coating or cover arranged around the heat conducting lead (320).

10. The delivery system (100) according to one of the preceding claims, **characterized in that** the delivery system (100) comprises a control device (400) for controlling a temperature of the connecting means (140) and/or the self-expanding structure (200), wherein the control device (400) is configured to control the cooling means (300).

11. The delivery system (100) according to claim 10, **characterized in that** the delivery system (100) comprises a first temperature sensor (401) for measuring a temperature of the connecting means (140), wherein the first temperature sensor (401) is configured to transmit a first measured temperature value (T1) to the control device (400), and wherein the delivery system (100) further comprises an additional sensor for measuring a heat flow and which is configured to transmit a first measured heat flow (HF1) to the control device (400), and wherein the control device (400) is configured to control the cooling means (300) using the first measured temperature value (T1) and the first measured heat flow (HF1) as an input, wherein particularly the first temperature sensor (401) is in direct thermal contact with the connecting means (140).

12. The delivery system (100) according to claim 11, **characterized in that** the delivery system (100) further comprises a second temperature sensor (402) for measuring a surrounding temperature, wherein the second temperature sensor (402) is configured to transmit a second measured temperature value (T2) to the control device (400), wherein the control device (400) is configured to control the cooling device (300) using the first measured temperature value (T1) and the second measured temperature value (T2) as an input and optionally using the first measured heat flow (HF1) as a further input, wherein particularly the second temperature sensor (402) is arranged at a tip section (130) at a distal end of the delivery system (100).

13. The delivery system (100) according to one of the claims 10 to 12, **characterized in that** the delivery system (100) comprises a recapture mechanism (150) configured to initiate recapture of the self-expanding structure (200) between the inner shaft (110) and the outer shaft (120) and/or the capsule (121), wherein the control device (400) is configured to automatically activate the cooling means (300) when the recapture mechanism (150) is activated.

14. The delivery system (100) according to one of the claims 10 to 13, **characterized in that** the delivery system (100) further comprises a signaling device (404) configured to generate a signal when the self-expanding structure (200) or the connecting means (140) has reached the desired temperature, wherein particularly the control device (400) is further configured to store a number of recapture events that have occurred, a maximum allowable number of recapture events and/or an allowable number of remaining recapture events, wherein the signaling device (404) is configured to indicate the number of recapture events that have occurred and/or the allowable number of remaining recapture events.

15. The delivery system (100) according to one of the claims 10 to 14, **characterized in that** the delivery system (100) comprises a blocking mechanism (160) configured to prevent recapture of the self-expanding structure (200) between the inner shaft (110) and the outer shaft (120) and/or the capsule (121), wherein the control device (400) is configured to deactivate the blocking mechanism (160) when the self-expanding structure (200) or the connecting means (140) has reached the desired temperature, wherein particularly the blocking mechanism (160) is configured to be manually deactivated, wherein more particularly the signaling device (404) is configured to generate a warning signal when the blocking mechanism (160) is manually deactivated.
